# EUROPEAN PATENT APPLICATION

(11) **EP 4 751 657 A1**
(43) Date of publication of application: **03.06.2026**
(21) Application number: 25213381.4
(22) Date of filing: 04.11.2025
(51) Int. Cl.: A61B 8/02, A61B 5/024, A61B 8/08, A61B 8/00

(54) **ON-DEMAND ULTRASOUND IMAGING FOR MATERNAL FETAL MONITORS**

(30) Priority: 27.11.2024 US 202418961966
(71) Applicant: GE Precision Healthcare LLC, Waukesha, WI 53188 (US)
(72) Inventor: HALDER, Purbasha, Waukesha, 53188 (US); NAIK, Rajendra, Waukesha, 53188 (US); GAVADE, Sandesh, Waukesha, 53188 (US)
(74) Representative: Kilburn & Strode LLP

(57) **Abstract**

A maternal fetal monitor includes a display, a processing device, and a memory device. The memory device includes instructions that are executable by the processing device to display a cardiotocogram for a maternal patient and a fetus of the maternal patient. Additionally, the instructions are executable by the processing device to display, in response to a request, an ultrasound image based on a plurality of ultrasound signals that are generated by, and detected by, an ultrasound imaging probe that is in communication with the maternal fetal monitor.

## Description

### BACKGROUND

The present disclosure generally relates to maternal fetal monitors, and specifically to on-demand ultrasound imaging for a maternal fetal monitor.

Maternal fetal monitors can continuously monitor fetal heart rate and uterine activities of pregnant women during the antepartum, intrapartum and postpartum period. To monitor fetal heart rate, the maternal fetal monitors use non-invasive and invasive probes like Doppler ultrasounds transducers, electrocardiogram (ECG) sensors, and fetal scalp electrodes.

### SUMMARY

A maternal fetal monitor includes a display, a processing device, and a memory device. The memory device includes instructions that are executable by the processing device to display a cardiotocogram for a maternal patient and a fetus of the maternal patient based on signals from the non-invasive and invasive probes. Additionally, the instructions are executable by the processing device to display, in response to a request, an ultrasound image based on a plurality of ultrasound signals that are generated by, and detected by, an ultrasound imaging probe that is in communication with the maternal fetal monitor.

In one embodiment, the ultrasound imaging probe includes a combination Doppler ultrasound transducer and imager.

In one embodiment, the instructions are executable by the processor to generate the cardiotocogram.

In one embodiment, the instructions are executable by the processor to generate the ultrasound image.

In one embodiment, the user interface is configured to provide a three-dimensional (3D) ultrasound image in response to the selection on the maternal fetal monitor.

In one embodiment, the instructions are executable by the processor to generate the 3D ultrasound image of an abdomen of the maternal patient, the image comprising a visual element that indicates where to locate one or more Doppler ultrasound transducers that detect fetal heart rate.

In one embodiment, the instructions are executable by the processor to present a user interface. The user interface is configured to present a heat map representing uterine activity, the heat map being superimposed on the 3D image, on the display in response to a selection on the user interface.

In one embodiment, the maternal fetal monitor provides power for the ultrasound imaging probe via the mUSB port.

In one embodiment, each of the subset of the mUSB ports is configured to connect with a device that generates one or more elements of the cardiotocogram.

In one embodiment, the maternal fetal monitor signals the ultrasound imaging probe to generate ultrasound signals for the ultrasound image in response to a selection on the maternal fetal monitor.

In one embodiment, the instructions are executable by the processor to display a plurality of cardiotocograms for a plurality of fetuses.

In one embodiment, the maternal fetal monitor is configured to switch a view on the display between the cardiotocogram and the ultrasound image in response to a signal from an ultrasound imaging probe.

In one embodiment, the instructions are executable by the processor to present a customizable on-screen keyboard for interacting with an ultrasound image.

In one embodiment, the instructions are executable by the processor to receive the ultrasound image from the ultrasound imaging probe.

A system includes an ultrasound imaging hub, an ultrasound imaging probe, and a maternal fetal monitor. The ultrasound imaging hub is in communication with the ultrasound imaging probe and the maternal fetal monitor. Additionally, the ultrasound imaging hub provides power for the ultrasound imaging probe. Further, the maternal fetal monitor is configured to display a cardiotocogram for a maternal patient and a fetus of the maternal patient, and display, in response to a request, an ultrasound image based on a plurality of ultrasound signals generated by the ultrasound imaging probe.

In one embodiment, the ultrasound imaging probe includes a combination Doppler ultrasound transducer and imagers.

In one embodiment, the maternal fetal monitor signals the ultrasound imaging probe to generate ultrasound signals for the ultrasound image in response to a selection on the display.

In one embodiment, the ultrasound imaging probe is configured to generate the ultrasound image.

A medical device includes a combination Doppler ultrasound transducer and imager. The combination Doppler ultrasound transducer and imager includes a first layer of piezoelectric crystals configured to generate first ultrasound signals at a first frequency, and detect first reflected signals of the first ultrasound signals. Additionally, the first reflected signals represent one or more cardiotocogram measures for a fetus of the maternal patient. Further, the combination Doppler ultrasound transducer and imager includes a second layer of piezoelectric crystals configured to generate a second ultrasound signals at a second frequency, and detect second reflected signals of the second ultrasound signals. Additionally, the second reflected signals represent an ultrasound image of the fetus and the maternal patient. Further, the combination Doppler ultrasound transducer and imager includes a button that, when selected, is configured to generate the second ultrasound signals, and send, for a maternal fetal monitor comprising a display, signals corresponding to the second reflected signals, the signals representing the ultrasound image.

In one embodiment, the combination Doppler ultrasound transducer and imager is configured to compute the cardiotocogram measures based on the first reflected signals, compute the ultrasound image based on the second reflected signals, send the cardiotocogram measures for the maternal fetal monitor, and send the ultrasound image for the maternal fetal monitor.

### BRIEF DESCRIPTION OF THE DRAWINGS

The present disclosure is described with reference to the following Figures.
Fig. 1A is a diagram of a system for on-demand ultrasound imaging with a maternal fetal monitor according to one embodiment of the present disclosure.
Fig. 1B is a diagram of system for on-demand ultrasound imaging with a maternal fetal monitor according to one embodiment of the present disclosure.
Fig. 2A is a diagram of system for on-demand ultrasound imaging with a maternal fetal monitor according to one embodiment of the present disclosure.
Fig. 2B is a diagram of system for on-demand ultrasound imaging with a maternal fetal monitor according to one embodiment of the present disclosure.
Fig. 3A is a side view of a maternal fetal monitor for on-demand ultrasound imaging according to one embodiment of the present disclosure.
Fig. 3B is a side view of a maternal fetal monitor for on-demand ultrasound imaging according to one embodiment of the present disclosure.
Fig. 3C is side view of a maternal fetal monitor for on-demand ultrasound imaging according to one embodiment of the present disclosure.
Fig. 3D is a side view of a maternal fetal monitor and external keyboard for on-demand ultrasound imaging according to one embodiment of the present disclosure.
Fig. 3E is a side view of a maternal fetal monitor for on-demand ultrasound imaging according to one embodiment of the present disclosure.
Fig. 3F is a side view of a maternal fetal monitor for on-demand ultrasound imaging according to one embodiment of the present disclosure.
Fig. 4 is an exemplary maternal fetal monitor with ultrasound imaging manager according to one embodiment of the present disclosure.

### DETAILED DESCRIPTION

In the present description, certain terms have been used for brevity, clarity and understanding. No unnecessary limitations are to be inferred therefrom beyond the requirement of the prior art because such terms are used for descriptive purposes only and are intended to be broadly construed.

As used herein, unless otherwise limited or defined, discussion of particular directions is provided by example only, with regard to particular embodiments or relevant illustrations. For example, discussion of "top," "bottom," "front," "rear," "left," "right," "horizontal," "vertical," and "longitudinal" features and/or relative motion, e.g., movement "up" and "down," is generally intended as a description only of the orientation of such features relative to a reference frame of a particular example or illustration. Correspondingly, for example, a "top" feature may sometimes be disposed below a "bottom" feature (and so on), in some arrangements or embodiments. Additionally or alternatively, embodiments may be arranged in a different orientation such that "top" and "bottom" features are arranged horizontally relative to each other, for example in a "left-to-right" orientation.

The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

As stated previously, a clinician or other medical professional may use a maternal fetal monitor to monitor fetal heart rate and uterine activity continuously over periods of time in the antepartum and intrapartum periods of pregnancy. Antepartum refers to the period of pregnancy preceding childbirth. Further, intrapartum refers to the time of childbirth, starting at the onset of labor. To determine fetal heart rate, the clinician may use a Doppler ultrasound transducer, fetal scalp electrodes (FSE), and/or electrodes capable of picking up electrocardio signals, just as in an ECG. To determine uterine activity, the clinician may use a tocodynamometer (Toco), intrauterine pressure catheter (IUPC), and/or electrodes that pick up electrical activity in the abdominal muscles (electromyography, EMG). These devices may generate a cardiotocogram (CTG) - a trace of the fetal heart rate and uterine contraction strengths over time - that the maternal fetal monitor may display. The CTG traces provide clinicians with useful information regarding the fetal wellbeing, as well as the progression of labor.

According to the American College of Obstetricians and Gynecologists (ACOG) and National Institute for Health and Care Excellence (NICE) guidelines, in cases of suspicious fetal heart rates, a medical professional may perform maternal repositioning, additional ultrasound imaging evaluation to reassess fetal health, and other actions. Also, ultrasound imaging is helpful when the clinician finds it difficult to locate the fetus, especially during multiple pregnancies or patients with a high body mass index (BMI). However, an ultrasound imaging probe may not be readily available. As such, performing ultrasound imaging may involve transporting the patient to another room with an ultrasound machine, or wheeling in a portable ultrasound machine into which the ultrasound imaging probe can connect to generate the images, e.g., into the labor and delivery room. This can lead to a delay in patient care or intervention. However, any delay at a time when fetal and/or maternal well-being may be at risk, can have catastrophic consequences, for the fetus and/or the pregnant patient.

Accordingly, the current inventors have endeavored to develop a maternal fetal monitor that adds ultrasound imaging capability to the maternal fetal monitor. According to some embodiments of the present disclosure, the maternal fetal monitor has a connection for an ultrasound imaging probe. Additionally, the maternal fetal monitor may receive signals from the ultrasound imaging probe, generate an ultrasound image from the received signals, and display the generated ultrasound image. Alternatively, the maternal fetal monitor 102A may connect with an ultrasound imaging probe having an active cable. In such an embodiment, the ultrasound imaging probe may acquire ultrasound signals, process these signals to generate an ultrasound image, and transmit the generated image to the maternal fetal monitor, which may display the ultrasound image as described below. Additionally, the maternal fetal monitor may include a user selectable mode to display images from an ultrasound scan by the ultrasound imaging probe. Thus, in response to a selection, the maternal fetal monitor may signal the ultrasound imaging probe to perform an ultrasound scan, and display the corresponding ultrasound image. According to some embodiments of the present disclosure, the ultrasound image display may be performed in a full screen mode, or on a portion of the screen, with the rest of the screen displaying other clinical data, such as the CTG data. Additionally, the maternal fetal monitor may perform these techniques with any remaining Doppler ultrasound transducers, Toco, IUPC, FSE or other probes still in place.

According to one embodiment of the present disclosure, the ultrasound imaging probe may be a conventional, ultrasound imaging probe having a layer of piezoelectric crystals (or arrays of piezoelectric crystals) that generate (analog) ultrasound signals, detect the reflections of the generated signals, and sends data representing the reflected signals to the maternal fetal monitor, which may generate the ultrasound image as described above. Alternatively, the ultrasound imaging probe may include a combination Doppler ultrasound transducer and imager, which may include the components of conventional maternal fetal monitor ultrasound transducer probes that work on the Doppler shift principle for FHR detection. Additionally, the combination Doppler ultrasound transducer and imager may include components for producing an ultrasound image. Accordingly, the combination Doppler ultrasound transducer and imager can be useful for monitoring fetal heart rate and/or producing an ultrasound image.

Advantageously, integrating the ultrasound imaging capability with the maternal fetal monitor may help clinicians, relatively quickly and accurately, locate the fetus to help place the Doppler ultrasound transducers for more accurate detection. Locating the fetus in this way may be beneficial in cases of multiple pregnancies, in patients whose BMI may interfere with identifying the fetus location, and other scenarios that may impede detection of one or more fetuses. Additionally, such embodiments may reduce the amount of time involved in ascertaining the health of the fetus(es) by eliminating the typical time used to transport the patient to an ultrasound machine(possibly in another ward), transport the ultrasound imaging machine to the patient, or switch from the maternal fetal monitor to a different machine altogether, having an ultrasound imaging probe and display. In these ways, embodiments of the present disclosure may improve patient outcomes by enabling clinicians to make more informed and timely decisions during the prepartum and intrapartum stages of pregnancy. Additionally, use of the same maternal fetal monitor for both cardiotocogram monitoring and ultrasound imaging may reduce the workflow by reducing the number of tasks involved, e.g., transporting the ultrasound imager, cleaning and disinfecting a display for the ultrasound imager. In these ways, such embodiments may additionally reduce risks to patient health, and costs for medical providers (and patients).

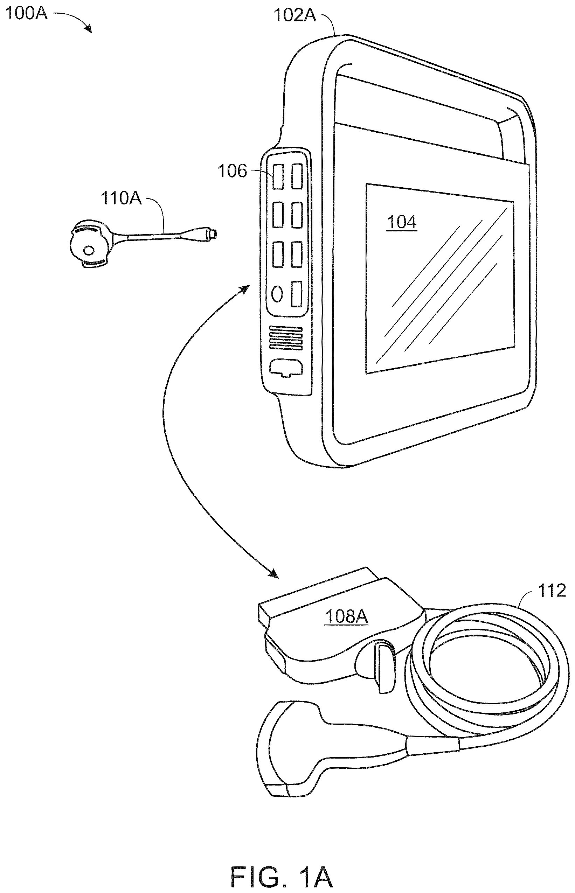

Fig. 1A is a diagram of a system 100A for on-demand ultrasound imaging with a maternal fetal monitor 102A according to one embodiment of the present disclosure. The system 100A includes the maternal fetal monitor 102A (having a display 104 and ports 106), ultrasound imaging probe 108A, and Doppler ultrasound transducer 110A. The display 104 may be an output device, or an input-output device, e.g., a touch screen. The ports 106 may be medical universal serial bus (mUSB) ports, which may be configured to connect with, and potentially power, multiple probes and/or sensors, such as the Toco, IUPC, FSE, EMG, ultrasound imaging probe 108A, and Doppler ultrasound transducer 110A. The maternal fetal monitor 102A may process signals from these probes and/or sensors to compute CTG measurements, generate ultrasound images, and the like. Accordingly, the maternal fetal monitor 102A may present the computed measurements and/or generated ultrasound images on the display 104. Alternatively, probes and/or sensors having active cables may compute the measurements and/or generate the ultrasound images, and provide the computed measurements and/or generated images for the maternal fetal monitor 102A to present on the display 104.

The ultrasound imaging probe 108A may be an electrically powered device that generates numerous ultrasound signals, and detects the reflections of these signals off of an area of study, e.g., the uterus of a pregnant woman. In some embodiments of the present disclosure, the ultrasound imaging probe 108A may be one of various types, such as, linear, convex, endocavitary, phased array, three-dimensional, four-dimensional, and the like. The ultrasound imaging probe 108A may connect to one of the ports 106 over a cable 112. The cable may be configured to exchange communications between the ultrasound imaging probe 108A and the maternal fetal monitor 102A. Thus, the ultrasound imaging probe 108A may send the analog representations of the signals that the ultrasound imaging probe 108A detects to the maternal fetal monitor 102A. Accordingly, the maternal fetal monitor 102A may convert the analog signals to digital signals representing an image, and display the image. Alternatively, the ultrasound imaging probe 108A may have an active cable. Having an active cable means that the ultrasound imaging probe 108A may be configured to process the analog signals and generate the ultrasound image. In such embodiments, the ultrasound imaging probe 108A may send the digital values of fetal heart rate and/or the ultrasound image to the maternal fetal monitor 102A.

The Doppler ultrasound transducer 110A may be an electrically powered device having a piezoelectric crystal layer, or array of piezoelectric crystals, that generates ultrasound signals, and detects the reflections of these signals off of the areas of study, and measures physiological information, such as the fetal heart rate, based on the Doppler shift in these reflected ultrasound signals. Further, the Doppler ultrasound transducer 110A may provide this physiological information for the maternal fetal monitor 102A, which may display the physiological information in a CTG.

According to some embodiments of the present disclosure, the maternal fetal monitor 102A may switch between a mode of continuous fetal monitoring (using the Doppler ultrasound transducer 110A), and a mode of ultrasound imaging (using the ultrasound imaging probe 108A). For example, a clinician may use the Doppler ultrasound transducer 110A to determine the fetal heart rate. Accordingly, the maternal fetal monitor 102A may present a CTG on the display 104 that shows the fetal heart rate over time based on signals that the Doppler ultrasound transducer 110A detects. However, the fetal heart rate presented on the display 104 may represent a condition under which an ultrasound imaging of the fetus is indicated, e.g., according to ACOG guidelines. Accordingly, the clinician may switch the mode of the maternal fetal monitor 102A to an imaging mode. According to one embodiment, switching to the imaging mode may involve making a touch selection on the display 104. In such an embodiment, the maternal fetal monitor 102A may signal the ultrasound imaging probe 108A to perform ultrasound imaging scanning, and to provide the detected signals or (in the case of an active cable), ultrasound image(s). Alternatively, the ultrasound imaging probe 108A may include a button that, in response to a selection, performs ultrasound imaging scanning, sends a request to the maternal fetal monitor 102A to switch to the ultrasound imaging mode, and sends signals representing the ultrasound image, or the ultrasound image itself to the maternal fetal monitor 102A. Accordingly, the maternal fetal monitor 102A may present the respective ultrasound image(s) on the display 104. According to some embodiments of the present disclosure, the maternal fetal monitor may present the ultrasound image(s) on the display 104 by itself, e.g., in a full screen mode, or with the CTG, e.g., in a split screen mode.

Alternatively, the maternal fetal monitor 102A may generate the ultrasound images. In such an embodiment, the ultrasound imaging probe 108A may provide data representing the reflected ultrasound signals for the maternal fetal monitor 102A, which may process this data to generate the ultrasound image(s). Further, the ultrasound imaging probe 108A may perform multiple ultrasound imaging scans, thus providing the ability to display a movie-like presentation of ultrasound images in real-time. Further, as described above, the ultrasound imaging probe 108A may provide signals that the maternal fetal monitor 102A processes to generate multiple ultrasound images. Alternatively, in the case of an ultrasound imaging probe having an active cable, the ultrasound imaging probe 108A may generate the multiple ultrasound images, and provide the generated ultrasound images for the maternal fetal monitor 102A to display.

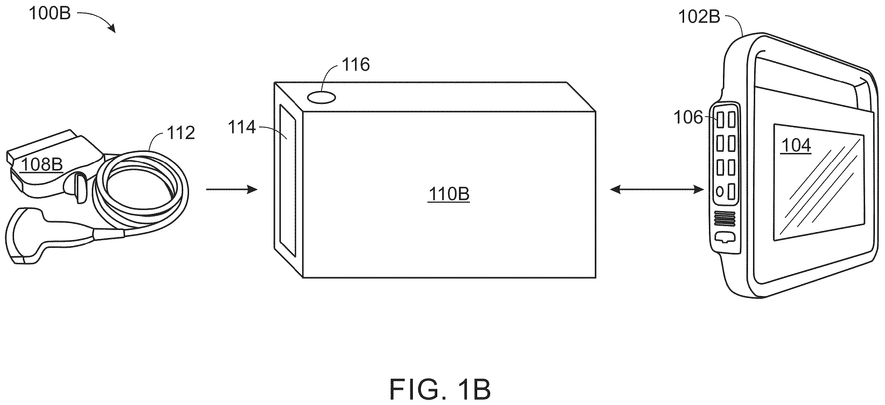

Fig. 1B is a diagram of system 100B for on-demand ultrasound imaging with a maternal fetal monitor 102B according to one embodiment of the present disclosure. The system 100B includes the maternal fetal monitor 102B (having display 104, ports 106), ultrasound imaging probe 108B, and ultrasound imaging hub 110B. The maternal fetal monitor 102B and ultrasound imaging probe 108B may be similar to maternal fetal monitor 102A and ultrasound imaging probe 108A, described with respect to Fig. 1A. However, in this example, the maternal fetal monitor 102B may not provide power to the ultrasound imaging probe 108B over mUSB ports. Instead, the ultrasound imaging probe 108B may connect with the ultrasound imaging hub 110B, which may provide power to the ultrasound imaging probe 108B over one or more hub ports 114. While the ultrasound imaging hub 110B shows one port 114, according to some embodiments, the ultrasound imaging hub 110B may include multiple ports 114, the number of which may vary. Further, the ports 114 may be configured for the connector of the ultrasound imaging probe 108B, an mUSB connector, an adaptor, and/or the like. Having multiple hub ports 114 may be useful for powering additional devices, i.e., an additional ultrasound imaging probe, a Toco, IUPC, and the like. In this example, the ultrasound imaging hub 110B includes a power button 116 that powers up the ultrasound imaging hub 110B (and connected devices) upon selection. However, some embodiments may not include the power button 116. Additionally, the ultrasound imaging hub 110B may connect with the maternal fetal monitor 102B via the ports 106. In this way, the ultrasound imaging hub 110B may exchange communications between the ultrasound imaging probe 108B and the maternal fetal monitor 102B. For example, the maternal fetal monitor 102B may receive a request via a touch-screen display to generate an ultrasound image. In response, the maternal fetal monitor 102 may provide the request for the ultrasound imaging hub 110B, which may provide the request for the ultrasound imaging probe 108B. Further, the ultrasound imaging probe 108B may perform ultrasound imaging scanning, and provide data representing the reflected ultrasound signals to the ultrasound imaging hub 110B, which may forward these signals to the maternal fetal monitor 102B. As stated previously, the maternal fetal monitor 102B may be configured to generate one or more ultrasound images from the signals, and present the image(s) on the display 104. Alternatively, where the ultrasound imaging probe 108B has an active cable, the ultrasound imaging probe 108B may generate the ultrasound image(s) based on the detected signals, and send the ultrasound image(s) to the ultrasound imaging hub 110B, which may send the ultrasound image(s) to the maternal fetal monitor 102B for presentation on the display 104.

As stated previously, the maternal fetal monitor 102B may present a CTG on the display 104 that shows the fetal heart rate over time. However, the fetal heart rate presented on the display 104 may represent a condition under which an ultrasound imaging of the fetus is indicated, e.g., per ACOG and/or NICE guidelines. Accordingly, the clinician may switch the mode of the maternal fetal monitor 102B to an imaging mode. According to one embodiment, switching to the imaging mode may involve making a touch selection on the display 104. Alternatively, the ultrasound imaging probe 108B may include a button that sends a request to the maternal fetal monitor 102B to switch to the imaging mode. Accordingly, the maternal fetal monitor 102B may present the ultrasound image(s) (generated by the ultrasound imaging probe 108B with an active cable, or generated by the maternal fetal monitor 102B) on the display 104. According to some embodiments of the present disclosure, the maternal fetal monitor 102B may present the ultrasound image(s) on the display 104 by itself, e.g., in a full screen mode, or with the CTG, e.g., in a split-screen mode.

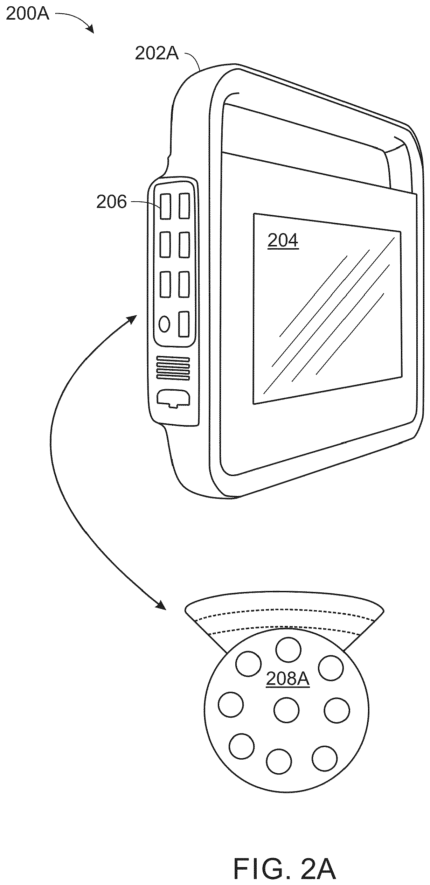

Fig. 2A is a diagram of system 200A for on-demand ultrasound imaging with a maternal fetal monitor 202A, according to one embodiment of the present disclosure. The system 200A includes the maternal fetal monitor 202A (having a display 204 and ports 206) and a combination Doppler ultrasound transducer and imager 208A. The maternal fetal monitor 202A may be similar to the maternal fetal monitor 102A, described with respect to Fig. 1A. The display 204, similar to display 104 described with respect to Figs. 1A, 1B, can be dynamically configured to show ultrasound images in predefined or user selectable screens. Alternatively, or additionally, the display can show the CTG. Further, the system 200A may include a conventional CTG device (not shown). In such embodiments, the maternal fetal monitor 202A may display both the ultrasound image and the CTG (having measurements from the convention CTG device). Similar to the maternal fetal monitor 102A, the maternal fetal monitor 202A can include multiple ports 206, similar to the ports 106, which can support active cables, and provide power for the combination Doppler ultrasound transducer and imager 208A.

According to some embodiments of the present disclosure, the combination Doppler ultrasound transducer and imager 208A may connect with the maternal fetal monitor 202A via ports 206, e.g., mUSB ports. Additionally, the combination Doppler ultrasound transducer and imager 208A may be similar to the Doppler ultrasound transducer 110A, with additional features for generating and detecting ultrasound signals that are useful for generating ultrasound images. More specifically, the combination Doppler ultrasound transducer and imager 208A may additionally include another layer of piezoelectric crystals (or arrays of piezoelectric crystals) for producing an ultrasound image. Further, the combination Doppler ultrasound transducer and imager may include a selection element (not shown) for performing either of a fetal heart rate scan, or ultrasound imaging scan. The combination Doppler ultrasound transducer and imager 208A may thus send CTG signals and/or ultrasound image signals to the maternal fetal monitor 202A for processing and display. Further, in combination devices having an active cable, the combination Doppler ultrasound transducer and imager 208A may compute the CTG measures, generate the ultrasound image, and send these to the maternal fetal monitor 202A for display. In these ways, the combination Doppler ultrasound transducer and imager 208A can be useful for monitoring fetal heart rate and/or producing an ultrasound image, depending on the mode that the clinician selects. Additionally, in the case of multiple pregnancies, the combination Doppler transducer and imager 208A may generate multiple ultrasound images. In such a case, the maternal fetal monitor 202A may present multiple images on the display 204 concurrently.

In this way, the combination Doppler ultrasound transducer and imager 208A provides a single device that can switch between continuous fetal monitoring and ultrasound imaging as clinically indicated in a continuous integrated workflow. More specifically, the convex surface side (e.g., the vertical side) of the combination Doppler ultrasound transducer and imager 208A includes the following layers: an innermost layer of damping material, an intermediate layer having piezoelectric crystal array, and an impedance matching layer having a material that matches or phases in/out the impedance. In other words, the impedance matching layer provides a gradual transition from the piezoelectric crystal to the skin, which reduces attenuation and increases efficiency. In these ways, these layers may enable the combination Doppler ultrasound transducer and imager 208A to act as a transducer for detecting fetal heart rate, and as an imaging ultrasound probe.

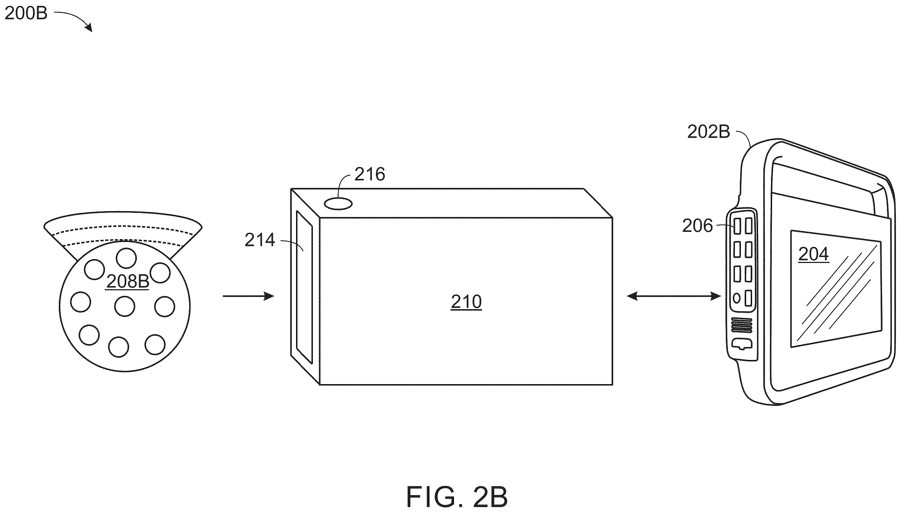

Fig. 2B is a diagram of a system 200B for on-demand ultrasound imaging with a maternal fetal monitor 202B according to one embodiment of the present disclosure. The system 200B includes the maternal fetal monitor 202B (having display 204 and ports 206), combination Doppler ultrasound transducer and imager 208B, and ultrasound imaging hub 210. The maternal fetal monitor 202B and combination Doppler ultrasound transducer and imager 208B are similar to the maternal fetal monitor 202A and combination Doppler ultrasound transducer and imager 208A, described with respect to Fig. 2A.

However, in this example, the maternal fetal monitor 202B may not provide power to the combination Doppler ultrasound transducer and imager 208B over mUSB ports. Instead, the combination Doppler ultrasound transducer and imager 208B may connect with the ultrasound imaging hub 210, which may be similar to the ultrasound imaging hub 110B, thus providing power to the combination Doppler ultrasound transducer and imager 208B over the hub port 214. The hub ports 214 may be similar to the hub ports 114, described with respect to Fig. 1B. As such, the ultrasound imaging hub 210 may include multiple hub ports 214 to enable the use of multiple combination Doppler ultrasound transducer and imagers 208A. In this example, the ultrasound imaging hub 210 includes a power button 216. However, some embodiments may not include the power button 216. Additionally, ultrasound imaging hub 210 may connect with the maternal fetal monitor 202B via the ports 206. In this way, the ultrasound imaging hub 210 may exchange communications between the combination Doppler ultrasound transducer and imager 208B and the maternal fetal monitor 202B. For example, the maternal fetal monitor 202B may receive a request via a touch-screen display to generate an ultrasound image. In response, the maternal fetal monitor 202B may provide the request for the ultrasound imaging hub 210, which may forward the request for the combination Doppler ultrasound transducer and imager 208B. Further, the combination Doppler ultrasound transducer and imager 208B may provide the signals detected by the combination Doppler ultrasound transducer and imager 208B to the ultrasound imaging hub 210, which may forward these signals to the maternal fetal monitor 202B. Alternatively, the combination Doppler ultrasound transducer and imager 208B may include a selection button or other actuator that switches the functionality of the combination Doppler ultrasound transducer and imager 208B between continuous Doppler monitoring and ultrasound imaging. Additionally, in the case of multiple pregnancies, the combination Doppler transducer and imager 208B may generate multiple ultrasound images. In such a case, the maternal fetal monitor 202B may present multiple images on the display 204 concurrently.

As stated previously, the maternal fetal monitor 202B may present a CTG on the display 204 that shows the fetal heart rate over time. However, the fetal heart rate presented on the display 204 may represent a condition under which an ultrasound imaging of the fetus is indicated, e.g., per ACOG and/or NICE guidelines. Accordingly, the clinician may switch the mode of the maternal fetal monitor 202B to an imaging mode. According to one embodiment, switching to the imaging mode may involve making a touch selection on the display 204. Accordingly, the maternal fetal monitor 202B may present the ultrasound image(s) on the display 204. According to some embodiments of the present disclosure, the maternal fetal monitor may present the ultrasound image(s) on the display 204 by itself, e.g., in a full screen mode, or with the CTG.

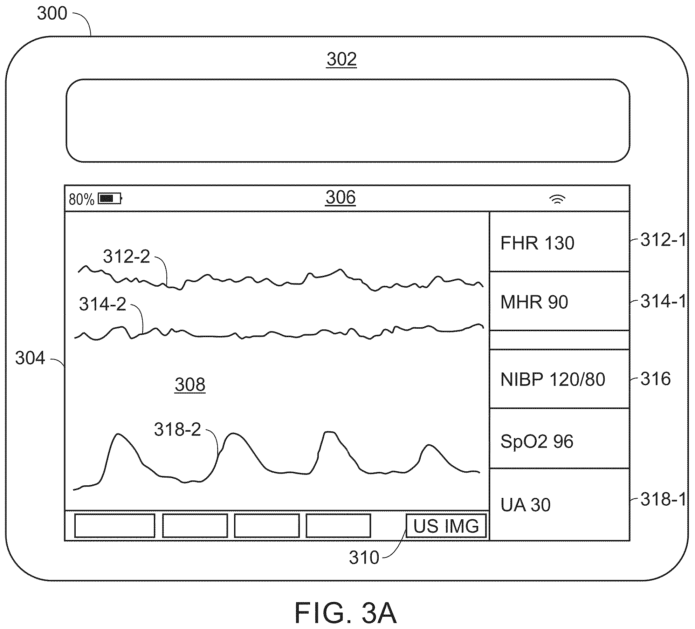

Fig. 3A is a side view of a maternal fetal monitor 300 for on-demand ultrasound imaging according to one embodiment of the present disclosure. The maternal fetal monitor 300 includes a handle 302 and display 304. The display 304 may be similar to the displays 104, 204 described with respect to Figs. 1A, 1B, 2A, and 2B. In this example, the display 304 is showing a status bar 306, a CTG view 308, and a display selection element 310A. In this example, the status bar 306 has indicators for battery power, e.g., 80%, and wireless network connectivity. However, the status bar 306 may have other like indicators not shown here. The CTG view 308 includes current fetal heart rate (FHR) 312-1, graphed FHR 312-2, current maternal heart rate (MHR) 314-1, graphed MHR 314-2, non-invasive blood pressure (NIBP) 316, specific percentage oxygen saturation, uterine activity 318-1, and graphed uterine activity 318-2. The current FHR 312-1 may represent the last FHR measurement. Further, the graphed FHR 312-2 may represent a series of the most recent FHR measurements over a specific time period. As stated previously, the Doppler ultrasound transducer 110A or combination Doppler ultrasound transducer and imager 208A, 208B may provide the FHR 312-1, 312-2 for presentation on the display 304. Similar to the FHR, the current MHR 314-1 and graphed MHR 314-2 1 may respectively represent the last MHR measurement, and a series of the most recent MHR measurements over the specific time period. Additionally, the NIBP 316 may represent the most recent measurement of maternal blood pressure. Further, the UA 318-1 may represent the current measurement of uterine activity of the maternal patient, and the graphed UA 318-2 may represent the uterine activity measurements over the specific time period.

According to some embodiments of the present disclosure, the display selection element 310 may be a user interface element that is selectable to change the display mode of the maternal fetal monitor 300 from a CTG display mode to an ultrasound image display mode. For example, a clinician or other operator may select the imaging selection element 310. In response, the maternal fetal monitor 300 may send a signal to the ultrasound imaging probe 108A, 108B, combination Doppler ultrasound transducer and imager 208A, 208B, and/or ultrasound imaging hub 110B, 210, to perform ultrasound imaging scanning and provide the analog signals representing the ultrasound image(s) to the maternal fetal monitor 300. Alternatively, for probes with active cables, the ultrasound imaging probes may generate the ultrasound image(s), and provide these image(s) to the maternal fetal monitor 300. Accordingly, the maternal fetal monitor 300 may present the ultrasound image(s) on the display 304.

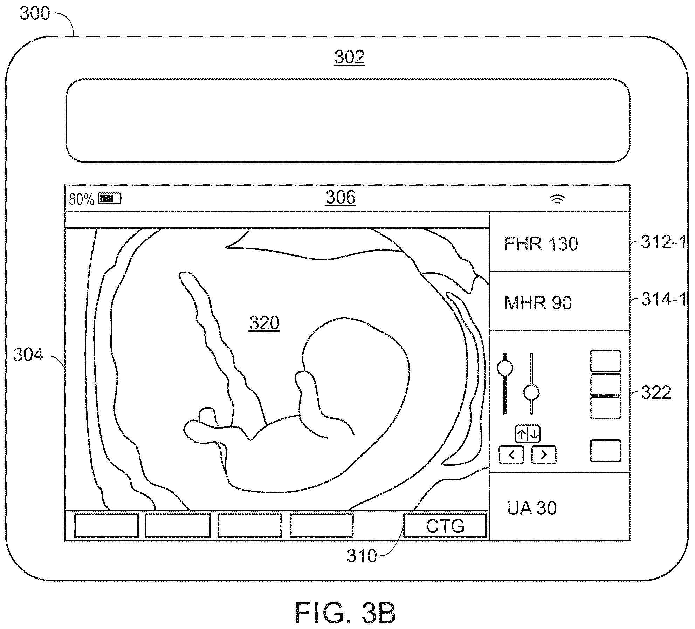

Fig. 3B is a side view of the maternal fetal monitor 300 for on-demand ultrasound imaging according to one embodiment of the present disclosure. The side view of Fig. 3B may represent the resultant display 304 after selection of the display selection element 310, described with respect to Fig. 3A. Accordingly, the display 304 shows an ultrasound image 320, and the CTG information (e.g., FHR 312-1, MHR 314-1) concurrently. Additionally, the display 304 is showing an image manipulation interface 322. The image manipulation interface 322 may enable the clinician or other operator to manipulate the display of the ultrasound image 320. More specifically, the image manipulation interface 322 may be useful for zooming in and out, rotating, and shifting the ultrasound image 320 in various directions and/or angles. Further, the display 304 is showing a display selection element 310, labelled, "CTG." According to some embodiments of the present disclosure, the maternal fetal monitor 300 may respond to a selection of the display selection element 310 by changing the presentation on the display 304 to the CTG display mode, as described with respect to Fig. 3A. Additionally, the maternal fetal monitor 300 may signal the ultrasound imaging probe 108A, 108B, combination Doppler ultrasound transducer and imager 208A, 208B, and/or ultrasound imaging hubs 110, 210 to stop ultrasound imaging functions.

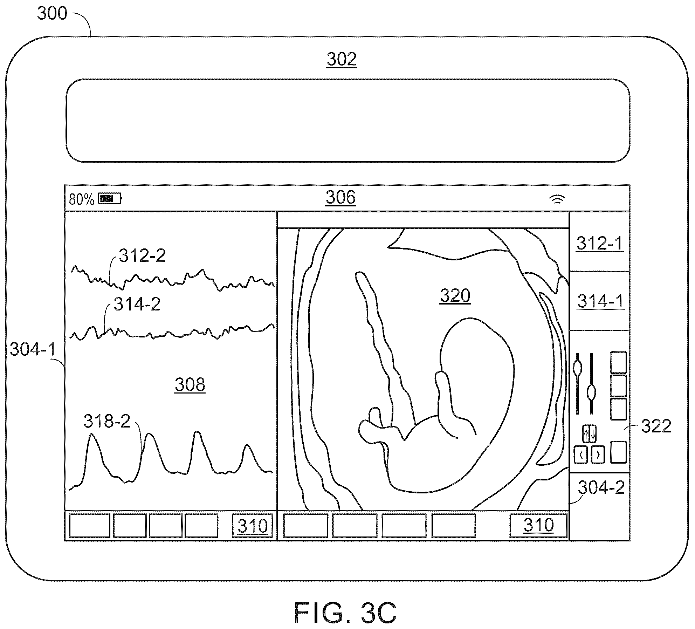

Fig. 3C is a side view of the maternal fetal monitor 300 for on-demand ultrasound imaging according to one embodiment of the present disclosure. In this example, there are two displays 304-1, 304-2, which may result from the use of two transducers connected directly or indirectly with the maternal fetal monitor 300, e.g., a Doppler ultrasound transducer 110A, 110B for display 304-1, and an ultrasound imaging probe 108A, 108B for display 304-2. Alternatively, the combination Doppler ultrasound transducer and imager 208A, 208B may be directly or indirectly connected with the maternal fetal monitor 300 to provide the signals and/or measurements for the display 304-1, and the signals and/or ultrasound image for the display 304-2. According to some embodiments of the present disclosure, the maternal fetal monitor 300 may automatically provide such a dual display in response to detecting multiple transducers connected with the maternal fetal monitor 300. Alternatively, the visual selection element 310 may provide selections for CTG display mode, ultrasound imaging display mode, and dual display mode.

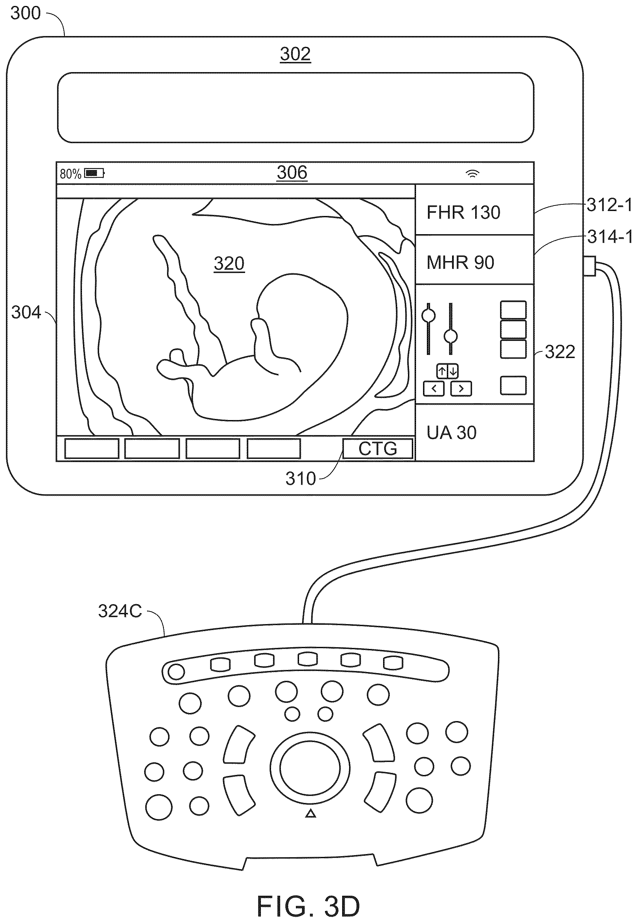

Fig. 3D is a side view of the maternal fetal monitor 300 and external keyboard 324-1 for on-demand ultrasound imaging according to one embodiment of the present disclosure. In this example, the external keyboard 324-1 is connected with the maternal fetal monitor 300. Accordingly, the clinician or operator may use the external keyboard 324C to operate the maternal fetal monitor 300, manipulate the ultrasound image 320, change the presentation mode on the display 304, and/or any other operations suitable for the operation of the maternal fetal monitor 300 and ultrasound imaging as described herein.

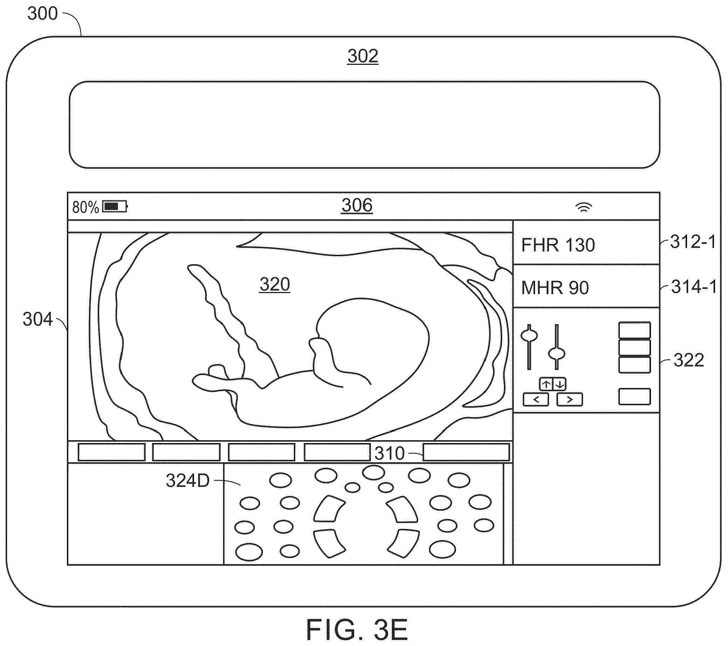

Fig. 3E is a side view of the maternal fetal monitor 300 for on-demand ultrasound imaging according to one embodiment of the present disclosure. In this example, the display includes an on-screen keyboard 324D. The on-screen keyboard 324D may be similar in functionality to the external keyboard 324C. However, the on-screen keyboard 324D may have more or fewer keys, depending on the available presentation space on the display 304. The maternal fetal monitor 300 may display the on-screen keyboard 324D automatically upon selection of the ultrasound imaging display mode. Alternatively, a clinician or other operator may select the on-screen keyboard 324C for display from a predetermined input selection list (not shown). Additionally, the clinician or other operator may configure the on-screen keyboard 324D, perform key selections, program keys, and the like, using on-screen selections (not shown).

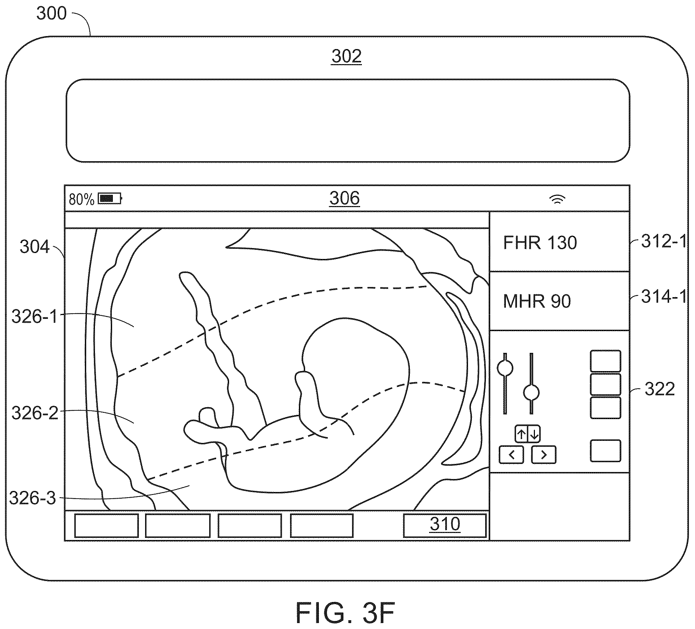

Fig. 3F is a side view of the maternal fetal monitor 300 for on-demand ultrasound imaging according to one embodiment of the present disclosure. In this example, the ultrasound image 320 includes a heat map that is superimposed on the ultrasound image of the contour of the abdomen. In this example, the heat map is represented as three zones 326 that may indicate varying levels of uterine activity. Additionally, the heat map may indicate where a clinician can place the Doppler ultrasound transducer probes, for example, in order to more effectively point in the direction of the fetal heart(s) and detect fetal heart rate. Additionally, the maternal fetal monitor 300 may be configured to automatically detect the location of the fetal heart, and provide an indication (not shown) on the display 304 of where to position the Doppler transducer(s). Further, the maternal fetal monitor 300 may be configured to determine the current stage of labor based on the heat map, and present this information on the display 304. The current stage of labor may describe the descent of the fetus for a given dilation of the cervix. For example, the maternal fetal monitor 300 may input the heat map to a machine learning model that is trained to determine the current stage of labor based on the heat map.

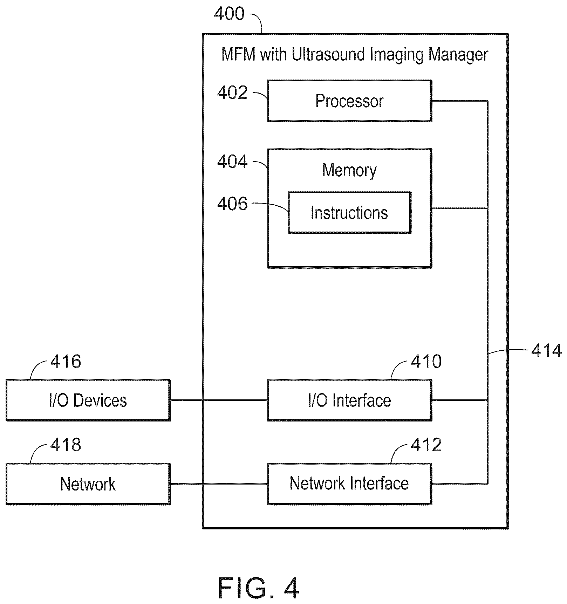

Fig. 4 is an exemplary maternal fetal monitor (MFM) with ultrasound imaging manager 400 according to one embodiment of the present disclosure. The example MFM with ultrasound imaging manager 400 may be similar to the maternal fetal monitors 102A, 102B, 202A, 202B, 300 described herein, and may perform on-demand ultrasound imaging as described with respect to Figs. 1A, 1B, 2A, 2B, and 3A through 3F. In this example, the MFM with ultrasound imaging manager 400 includes a processor 402, memory 404, input-output (I/O) interface 410, and network interface 412, which may be connected by an interconnect 414. The processor 402 may be a computer processing circuit (e.g., a central processing unit (CPU)) that retrieves and executes programming instructions 406 stored in the memory 404 to perform the functionality described herein. The interconnect 414 may move data, such as programming instructions, between the processor 402, memory 404, I/O interface 410, and network interface 412. The interconnect 414 may include one or more buses.

The memory 404 may be a computer memory or storage device, including volatile memory, such as a random access memory (RAM) device (e.g., static RAM, dynamic RAM, and the like), non-volatile memory, such as a hard disk drive, solid state device (SSD), removable memory cards, optical storage, flash memory devices, and the like. In some examples, the memory 404 may include volatile and non-volatile memory devices. Further, the memory 404 may store instructions 406, which may perform on-demand ultrasound imaging, as described with respect to Figs. 1A, 1B, 2A, 2B, and 3A through 3F.

Additionally, the MFM with ultrasound imaging manager 400 may be in electronic communication with I/O devices 416 through the I/O interface 410, and with a network 418 through the network interface 412. The I/O devices 416 may capture inputs and provide outputs as described herein, and may include ultrasound imaging hubs 110B, 210, ultrasound imaging probes 108A, 208A, Doppler ultrasound transducer 110A, and combination Doppler ultrasound transducers and imagers 208A, 208B. The network 418 may be an electronic communication network, such as a local area network, wide area network, and the like, for processing communications between the MFM with ultrasound imaging manager 400 and the machine learning models described herein. In some examples, the network 418 may be wired, wireless (e.g., wi-fi, Bluetooth, or cellular), or some other computer communication network.

In some embodiments, the MFM with ultrasound imaging manager 400 may be a server computer or similar device without a user interface but which receives requests from other computer systems having one or more user interfaces. Further, in some embodiments, the MFM with ultrasound imaging manager 400 may be a portable computer, laptop, tablet computer, pocket computer, telephone, smart phone, or the like.

It should be noted that, as used herein, the term mechanism can encompass hardware, software, firmware, or any suitable combination thereof. In some embodiments, any suitable computer readable media can be used for storing instructions for performing functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

This written description uses examples to disclose the invention(s), including the best mode, and also to enable any person skilled in the art to make and use the invention(s). Certain terms have been used for brevity, clarity, and understanding. No unnecessary limitations are to be inferred therefrom beyond the requirement of the prior art because such terms are used for descriptive purposes only and are intended to be broadly construed. The patentable scope of the invention(s) is defined by the claims and may include other examples that occur to those skilled in the art. Such other examples are intended to be within the scope of the claims if they have features or structural elements that do not differ from the literal language of the claims, or if they include equivalent features or structural elements with insubstantial differences from the literal languages of the claims.

## Claims

1. A maternal fetal monitor, comprising:
a display;
a processing device;
a memory device comprising instructions that are executable by the processor to:
display a cardiotocogram for a maternal patient and a fetus of the maternal patient; and
display, in response to a request, an ultrasound image based on a plurality of ultrasound signals that are generated by, and detected by, an ultrasound imaging probe that is in communication with to the maternal fetal monitor.

2. The maternal fetal monitor of claim 1, wherein the ultrasound imaging probe comprises a combination Doppler ultrasound transducer and imager.

3. The maternal fetal monitor of claim 1, wherein the instructions are executable by the processor to generate the cardiotocogram.

4. The maternal fetal monitor of claim 1, wherein the instructions are executable by the processor to generate the ultrasound image.

5. The maternal fetal monitor of claim 1, wherein the user interface is configured to provide a three-dimensional (3D) ultrasound image in response to the selection on the maternal fetal monitor.

6. The maternal fetal monitor of claim 5, wherein the instructions are executable by the processor to generate the 3D ultrasound image of an abdomen of the maternal patient, the image comprising a visual element that indicates where to locate one or more Doppler ultrasound transducers that detect fetal heart rate.

7. The maternal fetal monitor of claim 6, wherein the instructions are executable by the processor to present a user interface, wherein the user interface is configured to present a heat map representing uterine activity, the heat map being superimposed on the 3D image, on the display in response to a selection on the user interface.

8. The maternal fetal monitor of claim 1, wherein the maternal fetal monitor provides power for the ultrasound imaging probe via the mUSB port.

9. The maternal fetal monitor of claim 1, wherein each of a subset of the plurality of mUSB ports is configured to connect with a device that generates one or more elements of the cardiotocogram.

10. The maternal fetal monitor of claim 1, wherein the maternal fetal monitor signals the ultrasound imaging probe to generate ultrasound signals for the ultrasound image in response to a selection on the maternal fetal monitor.

11. The maternal fetal monitor of claim 1, wherein the instructions are executable by the processor to display a plurality of cardiotocograms for a plurality of fetuses.

12. The maternal fetal monitor of claim 1, wherein the maternal fetal monitor is configured to switch a view on the display between the cardiotocogram and the ultrasound image in response to a signal from the ultrasound imaging probe.

13. The maternal fetal monitor of claim 1, wherein the instructions are executable by the processor to present a customizable on-screen keyboard for interacting with an ultrasound image.

14. The maternal fetal monitor of claim 1, wherein the instructions are executable by the processor to receive the ultrasound image from the ultrasound imaging probe.
